# EUROPÄISCHE PATENTSCHRIFT

(11) **EP 1 243 286 B1**
(45) Veröffentlichungstag und Bekanntmachung des Hinweises auf die Patenterteilung: **05.03.2014**
(21) Anmeldenummer: 02090116.1
(22) Anmeldetag: 19.03.2002
(51) Int. Cl.: A61N 1/05

(54) **Intravaskuläre Elektrodenleitung**
Intravascular electrode lead
Fil d'électrode intravasculaire

(30) Priorität: 21.03.2001 DE 10114725
(43) Veröffentlichungstag der Anmeldung: 25.09.2002
(73) Patentinhaber: BIOTRONIK SE & Co. KG, 12359 Berlin (DE)
(72) Erfinder: Kolberg, Gernot, 12043 Berlin (DE); Schaldach, Max, verstorben (DE)
(74) Vertreter: Lindner-Vogt, Karin L.

(56) Entgegenhaltungen:
- EP-A2- 1 129 745
- US-A- 4 414 986
- US-A- 5 170 802
- US-A- 6 033 397
- US-A- 6 129 750

## Beschreibung

Die Erfindung betrifft eine intravaskuläre Elektrodenleitung mit einer zur Fixierung in einem Blutgefäß geeigneten Formgebung.

Elektrodenleitungen, die beispielsweise in Blutgefäße eingeführt werden oder durch Blutgefäße hindurch in eine Kammer eines Herzens, sind grundsätzlich bekannt. Solche Elektrodenleitungen tragen in der Regel Elektroden, die dazu dienen, elektrische Impulse an die Elektrodenleitung umgebendes Körpergewebe abzugeben oder elektrische Signale vom Körpergewebe aufzunehmen. Bekannt sind beispielsweise Stimulationselektroden für Herzschrittmacher.

Es ist auch bekannt Elektrodenleitungen zweidimensional, beispielsweise schlangenförmig so zu verformen, dass die äußeren Bögen der schlangenförmig geformten Elektrodenleitung an den Wänden eines Gefäßes anliegen und so der Elektrodenleitung in dem Gefäß Halt geben, wie beispielsweise die US-Patente 4,374,527, 5,922,014 und 5,925,073 zeigen. Eine Elektrodenleitung, die dreidimensional so verformt ist, dass sie der Leitung Halt im Atrium eines Herzens verschafft, ist beispielsweise aus der US 5,995,876 bekannt. Die Elektrodenleitung ist dabei so verformt, dass Elektroden im Bereich des Atriums am Myokard anliegen.

Außerdem sind aus den US-Patenten 5,387,233 und 6,129,750 helixförmig gewundene Elektrodenleitungen bekannt, die zum Einführen in ein Blutgefäß, nämlich in den Koronarsinus, ausgebildet sind und mit den Helixwindungen an den Gefäßwänden des Koronarsinus anliegen.

Die vorliegende Erfindung bezieht sich in erster Linie auf intravaskuläre Elektrodenleitungen, also auf Elektrodenleitungen für längsgestreckte Blutgefäße wie Arterien oder Venen. Im Gegensatz zu beispielsweise Herzkammern mit ihren haltgebenden Ausbuchtungen ist die Aufgabe, Elektrodenleitungen in längsgestreckten Blutgefäßen einen Halt zu geben, eine andere. Die Wände der Blutgefäße sollen möglichst nicht verletzt werden, außerdem soll das Blutgefäß weiterhin für Blut durchlässig bleiben und nicht durch die Elektrodenleitung verstopft werden.

Ausgehend vom geschilderten Stand der Technik liegt der Erfindung die Aufgabe zugrunde, eine intravaskuläre Elektrodenleitung mit einer alternativen haltgebenden Gestaltung anzugeben.

Erfindungsgemäß wird diese Aufgabe durch eine Elektrodenleitung der eingangs genannten Art gelöst, die zumindest in einem Abschnitt eine dreidimensionale Formgebung besitzt und dort einen längsgestreckten Hohlraum einschließt, wobei die Elektrodenleitung wenigstens zwei Leitungs(teil)abschnitte mit bezüglich der Hohlraumlängsrichtung unterschiedlicher Steigungsrichtung bzw. unterschiedlichem Windungssinn der Elektrodenleitung aufweist.

Die aus den US-Patenten 5,387,233 und 6,129,750 bekannten Elektrodenleitungen sind wie die erfindungsgemäße Elektrodenleitung ebenfalls dreidimensional, nämlich helixförmig verformt, wobei die jeweils von der Elektrodenleitung gebildete Helix einen Hohlraum einschließt. Bei diesen bekannten Elektrodenleitungen besitzt die Elektrodenleitung im Bereich der Helix durchgehend die gleiche Steigungsrichtung bzw. den gleichen Windungssinn. Die erfindungsgemäße Elektrodenleitung unterscheidet sich von diesem Stand der Technik dadurch, dass die Steigungsrichtung bzw. der Windungssinn der Elektrodenleitung in dem dreidimensional verformten Abstand zumindest einmal wechselt. Hieraus ergeben sich insbesondere die im Folgenden noch zu erläuternden Fertigungsvorteile. Außerdem können Torsionseffekte, die bei den bekannten helixartig verformten Elektrodenleitungen beim Strecken oder Stauchen des Helixabschnitts auftreten, gezielt kompensiert werden.

Eine sehr einfache und daher bevorzugte Elektrodenleitung besitzt zwei helixartig geformte Leitungsabschnitte gegenläufiger Steigung. Bei solch einer Elektrodenleitung ergibt sich die unterschiedliche Steigungsrichtung somit aus dem gegenläufigen Drehsinn der zugrundeliegenden Helices. Solch eine Elektrodenleitung lässt sich in einfacher Weise herstellen, indem sie zunächst nach Art eines an einer Seite offenen Dreiecks geformt wird. Anschließend wird das auf diese Weise gebildete Dreieck um einen Zylinder gewickelt. Es ergibt sich eine Elektrodenleitung die einfach herzustellen ist, und in der Torsionskräfte beim Strecken oder Stauchen des verformten Leitungsabschnittes bestmöglich kompensiert werden.

In einer alternativen, bevorzugten Ausführungsform ist die Abwicklung der einen Hohlzylinder einschließenden Elektrodenleitung nicht dreieck- sondern Ω-förmig. Wesentliches Merkmal dieser Ω-Form ist eine Art negativer Steigung oder Hinterschneidung, wie sie in der anschließenden Figurenbeschreibung am Bild erläutert ist. Diese negative Steigung oder Hinterschneidung führt dazu, dass sich der Durchmesser des eingeschlossenen Hohlraums vergrößert, so dass sich die entsprechend geformte Elektrodenleitung bei Zugbelastung in einem hohlzylinderförmigen Blutgefäß verkeilt, da sich die Elektrodenleitung bei Zugbelastung stärker gegen die Gefäßwand drückt. Die Fixierung der Elektrodenleitung wird somit bei Zugbelastung stärker. Ist die Steigung der vorgeformten Leitungsabschnitte hingegen nur positiv - unabhängig vom Drehsinn der zugrundeliegenden Helix - verkleinert sich der eingeschlossene Hohlraum bei Zugbelastung, so dass es nicht zu der verkeilenden Wirkung kommt. Außer aus Drehsinn der jeweils zugrundeliegenden Helix kann sich eine unterschiedliche Steigungsrichtung somit alternativ oder zusätzlich auch daraus ergeben, dass die den Hohlraum einschließenden Leitungsabschnitte auch Abschnitte aufweisen, in denen die Erstreckungskomponente der Elektrodenleitung parallel zur Längsrichtung des eingeschlossenen Hohlraums unterschiedliche Vorzeichen aufweist, mithin eine negative oder positive Steigung.

Weitere vorteilhafte Ausführungsvarianten betreffen das Einbringen der genannten Elektrodenleitungsvarianten in ein Blutgefäß. Dieses wird dadurch erleichtert, dass die Elektrodenleitung beim Einführen möglichst gestreckt ist und erst wenn sie an ihrem Bestimmungsort angelangt ist ihre dreidimensionale Verformung annimmt. Eine Ausführungsvariante, die dies in vorteilhafter Weise ermöglicht, besteht in einer dreidimensional vorgeformten Elektrodenleitung, die durch Einführen eines Stiletts in das von der Elektrodenleitung eingeschlossene Lumen so gestreckt werden kann, dass sie leicht in Blutgefäße einzuführen ist.

In einer alternativen Ausführungsvariante ist die Elektrodenleitung biegeweich ausgeführt und besitzt ein Lumen. In dieses Lumen kann beispielsweise ein steuerbarer Führungsdraht eingesetzt werden, um so die Elektrodenleitung beim Einführen in ein Blutgefäß gezielt steuern zu können. Wenn die Elektrodenleitung am Bestimmungsort angelangt ist, kann in das Lumen anstelle des Steuerdrahtes beispielsweise ein dreidimensional vorgeformtes Stilett, beispielsweise in Form eines dreidimensional vorgeformten Federdrahtes, eingeführt werden. Dieser prägt der biegeweichen Elektrodenleitung dann seine Form auf.

Eine weitere Ausführungsvariante besteht in einer Elektrodenleitung, die eine versteifende Wendel aus elastischem Material besitzt, welche in einer Vielzahl von Windungen gebildet wird. Im Lumen dieser Elektrodenleitung ist eine Sehne vorgesehen, die mit ihrem distalen Ende an der Elektrodenleitung befestigt ist. Durch Zug an der Sehne ergibt sich auf diese Weise eine stauchende Kraft in der Elektrodenleitung. Die Wendel ist so ausgebildet, dass die Elektrodenleitung bei Zug auf der Sehne aus ihrer Längsrichtung ausbiegt und eine vorgegebene dreidimensionale Form annimmt. Beim Einsatz entsprechender Materialien kann diese Form fixiert werden, so dass die Elektrodenleitung die dreidimensionale Verformung beibehält, wenn der Zug über die Sehne nachlässt.

Eine wieder andere Ausführungsform zeichnet sich durch ein Memorymetallelement aus, welches beispielsweise eine an sich bekannte Titanlegierung aufweist, die bei Überschreiten einer Auslösetemperatur ihre Form von einer ersten zu einer zweiten Form verändert. Dieses Memorymetallelement ist so ausgeführt, dass seine erste Form einer im Wesentlichen gestreckten Elektrodenleitung entspricht, die ein einfaches Einführen der Elektrodenleitung erlaubt, während die zweite Form des Memorymetalls nach Überschreiten der Auslösetemperatur zu einer dreidimensional verformten Elektrodenleitung führt. Vorteilhafter Weise kann ein Heizelement zum Erwärmen des Memorymetallelementes auf die Auslösetemperatur vorgesehen sein, falls die Körpertemperatur nicht ausreicht, um die Auslösetemperatur zu erreichen. Falls die Körpertemperatur ausreicht, um die Auslösetemperatur zu erreichen, bei der das Memorymetall seine Form verändert, können Kühlmittel vorgesehen sein, alternativ können die Elektrodenleitungen auch im gekühlten Zustand eingeführt werden, so dass es sich während und nach dem Einführen in das Blutgefäß langsam erwärmt und schließlich die Auslösetemperatur erreicht.

Vorteilhafterweise trägt die Elektrodenleitung im Bereich ihrer dreidimensionalen Verformung wenigstens eine Elektrode derart, dass die Elektrode an der Wand eines jeweiligen Blutgefäßes anliegt.

Die Erfindung soll nun anhand von Ausführungsbeispielen mithilfe der Figuren näher erläutert werden. Von den Figuren zeigen die
- Figuren 1a und b:: eine erste Ausführungsvariante einer dreidimensional verformten Elektrodenleitung und eine Skizze, wie diese Ausführungsvariante erzeugt werden kann;
- Figuren 2a und b:: eine alternative Ausführungsvariante und eine Skizze wie die alternative Ausführungsvariante erzeugt werden kann;
- Figuren 3a bis d:: eine Ausführungsvariante einer Elektrodenleitung die durch Stauchen einer versteifenden Wendel verformbar ist;
- Figuren 4a und b:: einen Abschnitt einer biegeweichen Elektrodenleitung, die durch Einsetzen eines vorgeformten Stiletts verformbar ist; und
- Figuren 5a bis c:: eine dreidimensional vorgeformte Elektrodenleitung, die durch Einführen eines streckenden Stiletts gestreckt werden kann.

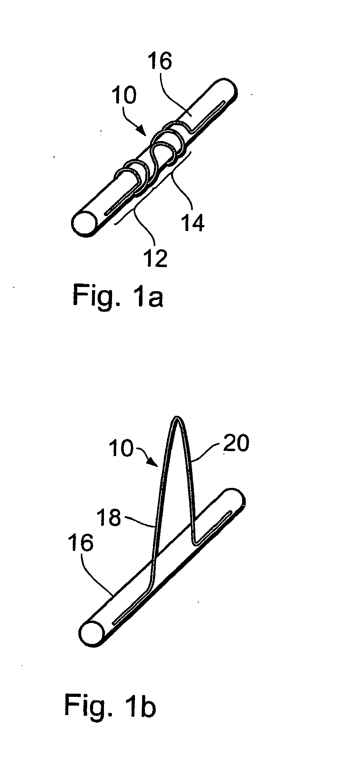

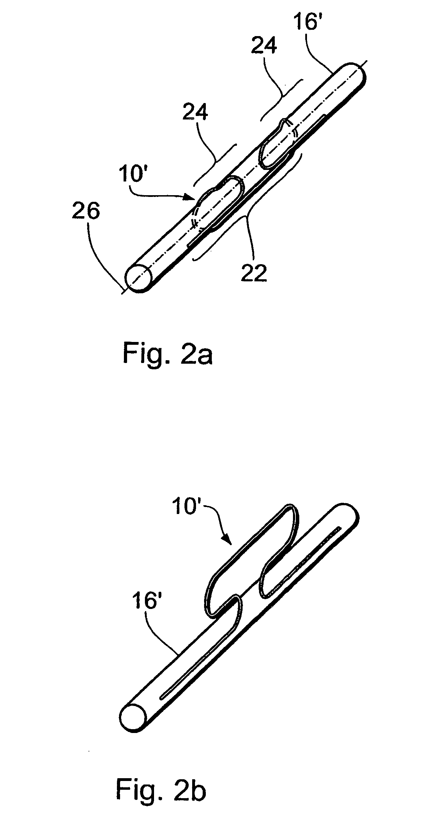

Die Figuren 1a und 2a zeigen jeweils skizzenhaft einen dreidimensional geformten Abschnitt einer Elektrodenleitung 10. Im Falle der Figur 1a setzt sich der dreidimensional geformte Abschnitt aus zwei Teilabschnitten 12 und 14 zusammen, die jeweils helixartig ausgebildet sind und sich durch die Steigung bzw. den Windungssinn der Helix unterscheiden. Der in Figur 1a dargestellte dreidimensional geformte Abschnitt der Elektrodenleitung kann dadurch hergestellt werden, dass die Elektrodenleitung 10 zunächst dreieckförmig geformt wird, wie in Figur 1b dargestellt ist. Anschließend wird der dreieckförmig geformte Abschnitt der Elektrodenleitung 10 um einen Zylinder 16 gewickelt, wie in Figur 1b angedeutet ist. Die zwei von jeweils einem Abschnitt 18 und 20 der Elektrodenleitung gebildeten Schenkel des Dreieckes ergeben auf diese Weise die beiden Teilabschnitte 12 und 14 der dreidimensional geformten Elektrodenleitung aus Figur 1a.

Der in Figur 2a abgebildete dreidimensional geformte Elektrodenabschnitt 22 kann auf ähnliche wie die zuvor beschriebene Weise hergestellt werden, indem die Elektrodenleitung 10' zunächst wie in Figur 2b angedeutet Ω-förmig vorgeformt wird und der Ω-förmige Abschnitt um einen Zylinder 16' gewickelt wird. Somit ist der dreidimensional geformte Leitungsabschnitt 22 der Elektrodenleitung 10' aus Figur 2a in der Abwicklung des von dem Leitungsabschnitt eingeschlossenen Zylinders Ω-förmig. Es ergeben sich somit Leitungsabschnitte 24, deren Erstreckungskomponenten parallel zu einer Längsachse 26 des eingeschlossenen Hohlraums 16' eine unterschiedliche Orientierung bzw. vorzeichenbehaftete Richtung, als die übrigen Leitungsabschnitte haben. Die Leitungsabschnitte 24 laufen "zurück", haben mithin eine negative Steigung und bewirken somit Hinterschneidungen.

Neben den angedeuteten Herstellungsvarianten sind auch andere denkbar. Die Figuren 1a und 1b sowie 2a und 2b dienen vor allem der Erläuterung des Zusammenhangs zwischen der jeweiligen dreidimensionalen Form der geformten Elektrodenleitung 10 bzw. 10' und der entsprechenden ebenen Darstellung durch Abwicklung des von der Elektrodenleitung eingeschlossenen Zylinders. Der von der Elektrodenleitung im dreidimensional geformten Abschnitt eingeschlossene Hohlraum muss nicht notwendigerweise zylinderförmig sein, er kann auch kegelstumpfförmig sein oder jede andere längsgestreckte Form, beispielsweise Prismenform, besitzen.

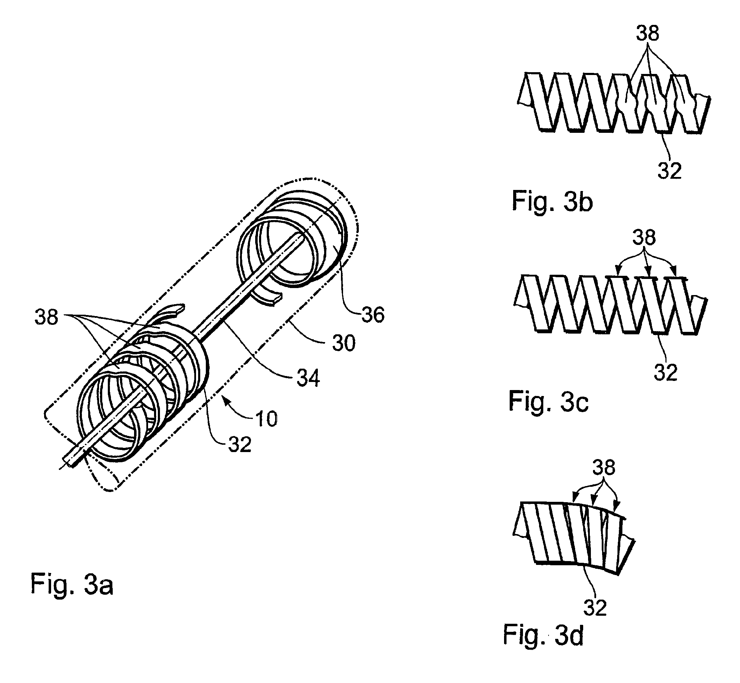

Die Figuren 3 bis 5 zeigen verschiedene Ausführungsvarianten von Elektrodenleitungen, die in gestreckter Form in ein jeweiliges Blutgefäß eingeführt werden können und nach dem Einführen ihre dreidimensionale Form annehmen können.

Die Elektrodenleitung 10 in Figur 3 umfasst eine Hülle 30 (in Figur 3a nur angedeutet) sowie innerhalb der Hülle 30 eine Metallwendel 32 und eine in einem von der Metallwendel 32 eingeschlossenen Lumen angeordnete Sehne 34 die an ihrem distalen Ende über eine Verbindungsplatte 36 mit der Metallwendel 32 verbunden ist.

Die Figur 3b zeigt einen Abschnitt der Metallwendel 32 in der Draufsicht; die Figur 3c den Abschnitt der Metallwendel 32 in der Seitenansicht. Zu erkennen ist, dass die einzelnen Windungen der Metallwendel 32 voneinander beabstandet sind und dass das Bandmaterial, aus dem die Metallwendel 32 besteht, an den Stellen 38 jeweils breiter ausgeführt ist. Durch Zug auf die Sehne 34 verkürzt sich die Metallwendel 32 bis die Windungen der Metallwendel 32 aneinander anliegen; siehe Figur 3d. Da das Bandmaterial der Metallwendel 32 an den Stellen 38 jeweils verbreitert ist, behält die verkürzte oder gestauchte Metallwendel 32 nicht ihre längsgestreckte Form, sondern nimmt die in Figur 3d dargestellte Biegung an. Nach dem in Figur 3 dargestellten Prinzip können die Wendeln entsprechend der Metallwendel 32 so gestaltet werden, dass eine Elektrodenleitung durch Zug auf eine eingebrachte Sehne beliebige dreidimensionale Krümmungen annimmt. Ohne Zug auf die Sehne ist die Elektrodenleitung gestreckt und biegeweich und kann leicht in das Blutgefäß eingeführt werden, wie dies in Figur 3a angedeutet ist.

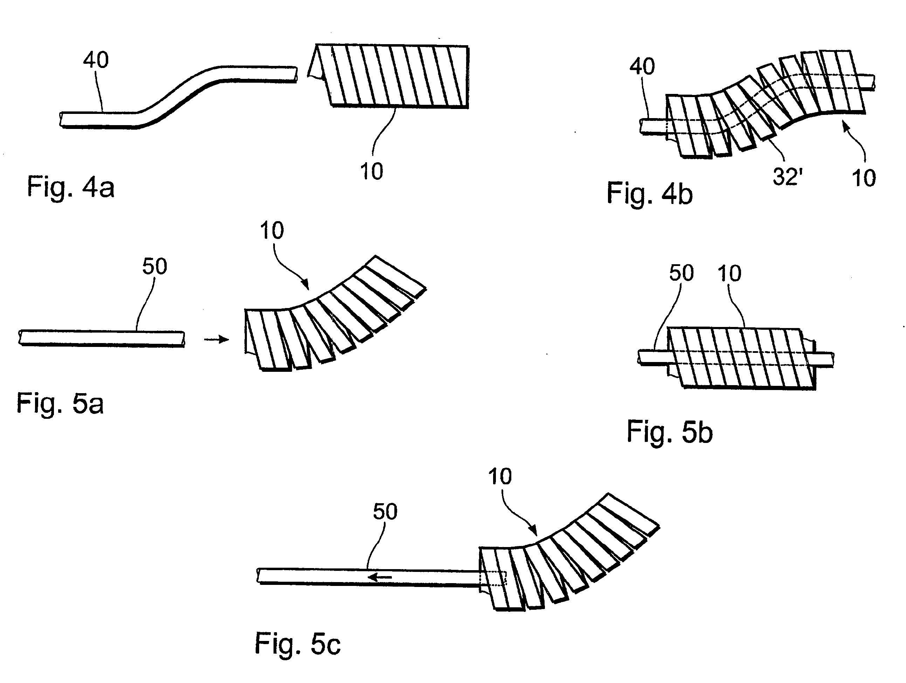

Den Figuren 4a und 4b liegt eine zunächst biegeweiche Elektrodenleitung 10 zugrunde, in die nach dem Platzieren in einem Blutgefäß ein vorgeformter Federstahldraht 40 nach Art eines Stiletts eingeführt werden kann, wie dies in Figur 4a angedeutet ist. Nach dem Einführen des Federstahldrahtes 40 nimmt die Elektrodenleitung 10 die durch den Federstahldraht 40 vorgegebene Form an; siehe Figur 4b.

Die Elektrodenleitung 10 aus den Figuren 5a und b ist als solches bereits dreidimensional vorgeformt und kann durch Einführen eines entsprechend steifen Stiletts 50 gestreckt werden, wie dies in Figur 5b angedeutet ist. Das Stilett 50 kann auch mäßig gebogen sein, um eine Steuerung des Endes der Elektrodenleitung 10 zu bewirken. Nach dem Platzieren der Elektrodenleitung 10 wird das Stilett 50 wieder entfernt, siehe Figur 5c, und die Elektrodenleitung 10 nimmt ihre ursprünglich vorgegebene, dreidimensionale Form an. Das Vorformen der Elektrodenleitung 10 aus Figur 5 geschieht, indem zunächst ein Draht ähnlich dem Federstahldraht 40 aus Figur 4 entsprechend der gewünschten Vorformung der Elektrodenleitung gebogen wird und anschließend die Metallwendel der Elektrodenleitung auf den vorgeformten Draht aufgeschoben wird. Ähnlich wie in Figur 4 nimmt die Metallwendel die Form des gebogenen Drahtes an. Anschließend wird die Metallwendel mitsamt dem gebogenen Draht bis zum Glühen erhitzt (geglüht), so dass sich das Gefüge der Metallwendel ändert und die Metallwendel auch ohne den gebogenen Draht ihre Vorform behält. Nach dem Glühen der Metallwendel kann diese auch Abgeschreckt, also schlagartig abgekühlt werden, so dass sich ein federelastisches Metallgefüge ergibt.

## Patentansprüche

1. Intravaskuläre Elektrodenleitung (10, 10'), mit mindestens einem zur Fixierung in einem Blutgefäß geeigneten Leitungsabschnitt (12, 14, 22), in dem die Elektrodenleitung dreidimensional so verformt ist, dass sie einen längsgestreckten Hohlraum einschließt, welcher ermöglicht, dass das Blutgefäß weiterhin für Blut durchlässig bleibt, **dadurch gekennzeichnet, dass** die Steigungsrichtung bzw. der Wicklungssinn der Elektrodenleitung im dreidimensional verformten Leitungsabschnitt zumindest einmal wechselt.

2. Elektrodenleitung nach Anspruch 1, **gekennzeichnet durch** zwei helixartig geformte Leitungsabschnitte (12, 14), die sich **durch** einen gegenläufigen Drehsinn in ihrer Steigungsrichtung voneinander unterscheiden.

3. Elektrodenleitung nach Anspruch 1 oder 2, **gekennzeichnet durch** zwei helixartig geformte Leitungsabschnitte, die sich, **durch** die Richtung einer parallel zu einer Längsachse des eingeschlossenen Hohlraums verlaufenden Erstreckungskomponente der Elektrodenleitung voneinander unterscheiden.

4. Elektrodenleitung nach Anspruch 1, **dadurch gekennzeichnet, dass** die Elektrodenleitung einen, einen Hohlzylinder einschließenden Abschnitt (22) aufweist, derart, dass die Leitung in einer abgewickelten Darstellung des Hohlzylinders f2-förmig ist.

5. Elektrodenleitung nach Anspruch 1, mit einem Lumen zum Einführen eines Steuermittels, **dadurch gekennzeichnet, dass** die Elektrodenleitung (10) dreidimensional vorgeformt und durch Einführen eines Stiletts in das Lumen streckbar ist.

6. Elektrodenleitung nach Anspruch 1, mit einem Lumen zum Einführen eines Steuermittels, **dadurch gekennzeichnet, dass** die Elektrodenleitung (10) biegeweich ist und durch Einführen eines vorgeformten Stiletts dreidimensional verformbar ist.

7. Elektrodenleitung nach Anspruch 1, mit einer Hülle, die eine versteifende Wendel (32) aus elastischem Material einschließt, die von einer Vielzahl von Windungen gebildet ist und ihrerseits ein Lumen einschließt, **dadurch gekennzeichnet, dass** im Lumen der Elektrodenleitung eine Sehne (34) derart angeordnet und mit einem distalen Ende der Sehne (34) befestigt ist, dass in der Elektrodenleitung (10) eine in Längsrichtung der Elektrodenleitung (10) wirkende, die Elektrodenleitung (10) stauchende Kraft erzeugbar ist und dass die Windungen der Wendel (32) bildende elastische Material derart geformt ist, dass sich die Elektrodenleitung bei angespannter Sehne (34) und Wirken der stauchenden Kraft dreidimensional verformt.

8. Elektrodenleitung nach Anspruch 1, **gekennzeichnet durch** ein Memorymetallelement, welches seine Form bei Überschreiten einer Sprungtemperatur von einer ersten vorgegebenen Form zu einer zweiten vorgegebenen Form verändert, wobei die erste Form des Memorymetallelementes mit einer im Wesentlichen gestreckten Elektrodenleitung korrespondiert und die zweite Form zu einer dreidimensional verformten Elektrodenleitung führt.

9. Elektrodenleitung nach Anspruch 8, **gekennzeichnet durch** ein Heizelement zum Heizen des Memorymetallelementes auf die Sprungtemperatur.

10. Elektrodenleitung nach Anspruch 1, die wenigstens eine Elektrode zum Aufnehmen und/oder Abgeben elektrischer Signale von oder an die Elektrodenleitung umgebendes Körpergewebe aufweist, **dadurch gekennzeichnet, dass** zumindest eine Elektrode im Bereich der dreidimensionalen Verformung der Elektrodenleitung angeordnet ist.

11. Verfahren zur Herstellung einer Elektrodenleitung nach den Ansprüchen 1 bis 3 und 5 bis 10, **dadurch gekennzeichnet, dass** die Elektrodenleitung (10) zunächst dreieckförmig geformt wird, und anschließend der dreieckförmig geformte Abschnitt der Elektrodenleitung um einen Zylinder (16) gewickelt wird, so dass die zwei von jeweils einem Abschnitt (18 und 20) der Elektrodenleitung gebildeten Schenkel des Dreieckes die beiden Teilabschnitte (12 und 14) der dreidimensional geformten Elektrodenleitung bilden.

12. Verfahren zur Herstellung einer Elektrodenleitung nach den Ansprüchen 4 bis 10, **dadurch gekennzeichnet, dass** die Elektrodenleitung (10') i2-förmig vorgeformt wird und der Ω-förmig Abschnitt um einen Zylinder (16') gewickelt wird, so dass der dreidimensional geformte Leitungsabschnitt (22) der Elektrodenleitung in der Abwicklung des von dem Leitungsabschnitt eingeschlossenen Zylinders Ω-förmig ist.

## Claims

1. An intravascular electrode line (10, 10'), comprising at least one line portion (12, 14, 22) which is suitable for fixing in a blood vessel, in which the electrode line is deformed three-dimensionally such that it encloses an elongate cavity, which makes it possible for blood to still flow through the blood vessel, **characterised in that** the pitch direction or the winding direction of the electrode line in the three-dimensionally formed line portion changes at least once.

2. The electrode line according to Claim 1, **characterised by** two helically formed line portions (12, 14) which differ from one another by an opposed direction of rotation in their pitch direction.

3. The electrode line according to Claim 1 or 2, **characterised by** two helically formed line portions which differ from one another by the direction of an extension component of the electrode line, said extension component running parallel to a longitudinal axis of the enclosed cavity.

4. The electrode line according to Claim 1, **characterised in that** the electrode line comprises a portion (22) enclosing a hollow cylinder in such a way that the line is Ω-shaped in an unwound illustration of the hollow cylinder.

5. The electrode line according to Claim 1, comprising a lumen for insertion of a control means, **characterised in that** the electrode line (10) is three-dimensionally deformed and can be stretched by inserting a stiletto into the lumen.

6. The electrode line according to Claim 1, comprising a lumen for inserting a control means, **characterised in that** the electrode line (10) is flexible and can be deformed three-dimensionally by insertion of a pre-shaped stiletto.

7. The electrode line according to Claim 1, comprising a sleeve which encloses a stiffening coil (32) made of resilient material that is formed by a plurality of windings and in turn encloses a lumen, **characterised in that** a filament (34) is arranged in the lumen of the electrode line and is secured at a distal end of the filament (34) in such a way that a force acting in the longitudinal direction of the electrode line (10) and compressing the electrode line (10) can be generated in the electrode line (10), and **in that** the resilient material forming the turns of the coil (32) is formed in such a way that the electrode line is deformed three-dimensionally when the filament (34) is tensioned and the compressing force is effective.

8. The electrode line according to Claim 1, **characterised by** a memory metal element which, when a transition temperature is exceeded, changes its form from a first predefined form to a second predefined form, wherein the first form of the memory metal element corresponds to a substantially stretched electrode line and the second form leads to a three-dimensionally deformed electrode line.

9. The electrode line according to Claim 8, **characterised by** a heating element for heating the memory metal element to the transition temperature.

10. The electrode line according to Claim 1, comprising at least one electrode for receiving and/or delivering electric signals from or to the bodily tissue surrounding the electrode line, **characterised in that** at least one electrode is arranged in the region of the three-dimensional deformation of the electrode line.

11. A method for producing an electrode line according to Claims 1 to 3 and 5 to 10, **characterised in that** the electrode line (10) is initially formed in a triangular manner and the triangularly formed portion of the electrode line is then wound around a cylinder (16), such that the two branches of the triangle each formed by a portion (18 and 20) of the electrode line form the two portions (12 and 14) of the three-dimensionally formed electrode line.

12. A method for producing an electrode line according to Claims 4 to 10, **characterised in that** the electrode line (10') is preformed in an Ω-shaped manner and the Ω-shaped portion is wound around a cylinder (16'), such that the three-dimensionally formed line portion (22) of the electrode line is Ω-shaped in the winding-up of the cylinder enclosed by the line portion.

## Revendications

1. Ligne d'électrodes intravasculaire (10, 10') avec au moins une section de ligne (12, 14, 22) appropriée pour la fixation dans un vaisseau sanguin, dans laquelle la ligne d'électrodes est déformée de façon tridimensionnelle, de manière à confiner un espace creux allongé permettant au vaisseau sanguin de continuer à laisser passer du sang, **caractérisée en ce que** la direction d'inclinaison ou le sens d'enroulement de la ligne d'électrodes varie au moins une fois dans la section de ligne déformée de façon tridimensionnelle.

2. Ligne d'électrodes selon la revendication 1, **caractérisée par** deux sections de ligne hélicoïdales (12, 14) qui se différencient l'une de l'autre par un sens de rotation inversé dans leur direction d'inclinaison.

3. Ligne d'électrodes selon la revendication 1 ou 2, **caractérisée par** deux sections de ligne hélicoïdales qui se distinguent l'une de l'autre par la direction d'un composant d'allongement de la ligne d'électrodes s'étendant parallèlement à un axe longitudinal de l'espace creux confiné.

4. Ligne d'électrodes selon la revendication 1, **caractérisée en ce que** la ligne d'électrodes comporte une section (22) renfermant un cylindre creux, de sorte que la ligne présente une forme en Ω dans une représentation déroulée du cylindre creux.

5. Ligne d'électrodes selon la revendication 1, avec un lumen pour l'insertion d'un moyen de commande, **caractérisée en ce que** la ligne d'électrodes (10) est préformée de façon tridimensionnelle et peut être étirée dans le lumen en insérant un stylet.

6. Ligne d'électrodes selon la revendication 1, avec un lumen pour l'insertion d'un moyen de commande, **caractérisée en ce que** la ligne d'électrodes est souple et flexible et peut être déformée de façon tridimensionnelle en insérant un stylet préformé.

7. Ligne d'électrodes selon la revendication 1, avec une gaine renfermant une hélice rigidifiante (32) constituée d'un matériau élastique, laquelle est formée par une pluralité de spires et renferme quant à elle un lumen, **caractérisée en ce qu'**un tendon (34) est disposé de telle façon dans le lumen de la ligne d'électrodes et fixé par une extrémité distale du tendon (34), qu'une force agissant dans le sens longitudinal de la ligne d'électrodes (10) et refoulant la ligne d'électrodes (10) peut être produite dans la ligne d'électrodes (10), et **en ce que** le matériau élastique formant les spires de l'hélice (32) est formé de telle façon que la ligne d'électrodes se déforme de façon tridimensionnelle lorsque le tendon (34) est tendu et la force de refoulement agit.

8. Ligne d'électrodes selon la revendication 1, **caractérisée par** un élément métallique à mémoire de forme modifiant sa forme lors du dépassement d'une température de changement, d'une première forme prédéfinie à une deuxième forme prédéfinie, la première forme de l'élément métallique à mémoire de forme correspondant à une ligne d'électrodes essentiellement étirée et la deuxième forme donnant une ligne d'électrodes déformée de façon tridimensionnelle.

9. Ligne d'électrodes selon la revendication 9, **caractérisée par** un élément de chauffage pour chauffer l'élément métallique à mémoire de forme à la température de changement.

10. Ligne d'électrodes selon la revendication 1, comportant au moins une électrode pour la réception et/ou l'émission de signaux électriques venant d'un tissu corporel entourant la ligne d'électrodes ou s'adressant à celui-ci, **caractérisée en ce qu'**au moins une électrode est installée dans la région de la déformation tridimensionnelle de la ligne d'électrodes.

11. Procédé pour la fabrication d'une ligne d'électrodes selon les revendications 1 à 3 et 5 à 10, **caractérisée en ce que** la ligne d'électrodes (10) est tout d'abord formée en forme de triangle, puis la section formée en forme de triangle de la ligne d'électrodes est enroulée autour d'un cylindre (16), de manière à ce que les deux jambes du triangle formées respectivement par une section (18 et 20) de la ligne d'électrodes forment les deux sections partielles (12 et 14) de la ligne d'électrodes formée de façon tridimensionnelle.

12. Procédé pour la fabrication d'une ligne d'électrodes selon les revendications 4 à 10, **caractérisée en ce que** la ligne d'électrodes (10') est préformée en forme de Ω, et la section en forme de Ω est enroulée autour d'un cylindre (16'), de telle sorte que la section de ligne (22) formée de façon tridimensionnelle de la ligne d'électrodes présente une forme en Ω dans le déroulement du cylindre enfermé dans la section de ligne.
